# EUROPEAN PATENT SPECIFICATION

(11) **EP 3 555 565 B1**
(45) Date of publication and mention of the grant of the patent: **08.09.2021**
(21) Application number: 17826400.8
(22) Date of filing: 18.12.2017
(51) Int. Cl.: G01C 21/00, A61B 5/06, G01R 33/00, G01R 33/09

(54) **DOMINANT AXIS NAVIGATION SENSOR**
NAVIGATIONSSENSOR MIT DOMINANTER ACHSE
CAPTEUR DE NAVIGATION À AXE DIRECTEUR

(30) Priority: 19.12.2016 US 201662436418 P
(43) Date of publication of application: 23.10.2019
(73) Proprietor: Boston Scientific Scimed Inc., Maple Grove, Minnesota 55311 (US)
(72) Inventor: HEIN, Matthew, Eden Prairie, Minnesota 55346 (US); FOSTER, Daniel J., Lino Lakes, Minnesota 55038 (US); VISWANATHAN, Raju, Mountain View, California 94041 (US)
(74) Representative: Pfenning, Meinig & Partner mbB
(86) International application number: PCT/US2017/067111
(87) International publication number: WO 2018/118814

(56) References cited:
- WO-A1-2016/087970
- WO-A1-2016/196985
- US-B1- 6 484 118

## Description

### CROSS-REFERENCE TO RELATED APPLICATION

This application claims priority to Provisional Application No. 62/436,418, filed December 19, 2016.

### TECHNICAL FIELD

The present disclosure relates to systems, methods, and devices for tracking items. More specifically, the disclosure relates to systems, methods, and devices for electro-magnetically tracking medical devices used in medical procedures.

### BACKGROUND

A variety of systems, methods, and devices can be used to track medical devices. Tracking systems can use externally generated magnetic fields that are sensed by at least one tracking sensor in the tracked medical device. The externally generated magnetic fields provide a fixed frame of reference, and the tracking sensor senses the magnetic fields to determine the location and orientation of the sensor in relation to the fixed frame of reference. Documents WO 2016/087970 A1, US 6,484,118 B1 and WO 2016/196985 A1 relate to various medical devices including position sensors.

### SUMMARY

The invention is defined by the independent claims. Preferred embodiments are set out in the dependent claims.

A sensor assembly in accordance with the invention includes a first magnetic field sensor and a second magnetic field sensor. The first magnetic field sensor includes a first substrate and a first elongated magnetic field sensor component extending a first length. The second magnetic field sensor includes a second substrate and a second elongated magnetic field sensor component extending a second length that is shorter than the first length. The first magnetic field sensor has a greater sensitivity to a sensed magnetic field than the second magnetic field sensor. The first elongated magnetic field sensor component is first flux guide, and wherein the second elongated magnetic field sensor is a second flux guide. The first magnetic field sensor includes a first magneto-resistive (MR) sensing element positioned adjacent the first flux guide, and wherein the second magnetic field sensor includes a second MR sensing element positioned adjacent the second flux guide.

In an embodiment, the first and second MR sensing elements are one of giant-magneto-resistive sensing elements, tunneling-magneto-resistive sensing elements, hall-effect sensing elements, colossal magneto-resistive sensing elements, extraordinary magneto-resistive sensing elements, and spin hall sensing elements.

Furthermore, optionally, the first elongated magnetic field sensor component is a first anisotropic-magneto-resistive (AMR) sensing element, wherein the second elongated magnetic field sensor component is a second AMR sensing element.

In an embodiment, the first magnetic field sensor is configured to sense a first component of a magnetic field along a first axis, wherein the second magnetic field sensor is configured to sense a second component of the magnetic field along a second axis.

In an embodiment, the first magnetic field sensor and the second magnetic field sensor are coupled to a common substrate.

In an embodiment, the sensor assembly further includes a third magnetic field sensor including a third substrate and a third elongated magnetic field sensor component extending a third length that is shorter than the first length.

In an embodiment, the first magnetic field sensor is at least twice as sensitive as the second magnetic field sensor.

In an embodiment, a system includes the inventive sensor assembly and a magnetic field generator configured to generate a magnetic field and a probe having a longitudinal axis and with the sensor assembly positioned at or near a distal end of the probe.

In an embodiment, the first magnetic field sensor of the system is configured to sense a first component of the magnetic field along the longitudinal axis, while the second magnetic field sensor is configured to sense a second component of the magnetic field along a second axis, and wherein the first magnetic sensor has a greater sensitivity to the first component of the magnetic field than a sensitivity of the second magnetic field sensor with respect to the second component of the magnetic field.

In an embodiment of the system, the first magnetic field sensor includes a first magneto-resistive (MR) sensing element, wherein the second magnetic field sensor includes a second MR sensing element, wherein the first MR sensing element is longer than the second MR sensing element.

Optionally, the first flux guide may be longer than the second flux guide.

In an embodiment of the system, the first magnetic field sensor includes a first magneto-resistive (MR) sensing element, wherein the second magnetic field sensor includes a second MR sensing element, and wherein the first and second magnetic field sensors are one of giant-magneto-resistive sensing elements, tunneling-magneto-resistive sensing elements, hall-effect sensing elements, colossal magneto-resistive sensing elements, extraordinary magneto-resistive sensing elements, and spin hall sensing elements.

In an embodiment, the system further includes a third magnetic field sensor configured to sense a third component of the magnetic field along a third axis that is different than the longitudinal axis and the second axis.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 shows a schematic of a tracking system, in accordance with certain embodiments of the present disclosure.
FIG. 2 shows a schematic of a sensor assembly, in accordance with certain embodiments of the present disclosure.
FIG. 3 shows a cut-away schematic of a probe, in accordance with certain embodiments of the present disclosure.
FIG. 4 shows a cut-away schematic of a probe, in accordance with certain embodiments of the present disclosure.
FIG. 5 shows a cut-away schematic of a probe, in accordance with certain embodiments of the present disclosure.
FIG. 6 shows a cut-away schematic of a probe, in accordance with certain embodiments of the present disclosure.

While the disclosure is amenable to various modifications and alternative forms, specific embodiments have been shown by way of example in the drawings and are described in detail below. The intention, however, is not to limit the disclosure to the particular embodiments described.

### DETAILED DESCRIPTION

During medical procedures, medical devices such as probes (e.g., catheters) are inserted into a patient through the patient's vascular system and/or a catheter lumen. To track the location and orientation of a probe within the patient, probes can be provisioned with magnetic field sensors. As probes become smaller and/or carry more components, the space or real estate available for magnetic field sensors decreases. Certain embodiments of the present disclosure are accordingly directed to systems, methods, and devices including sensor assemblies with compact geometry.

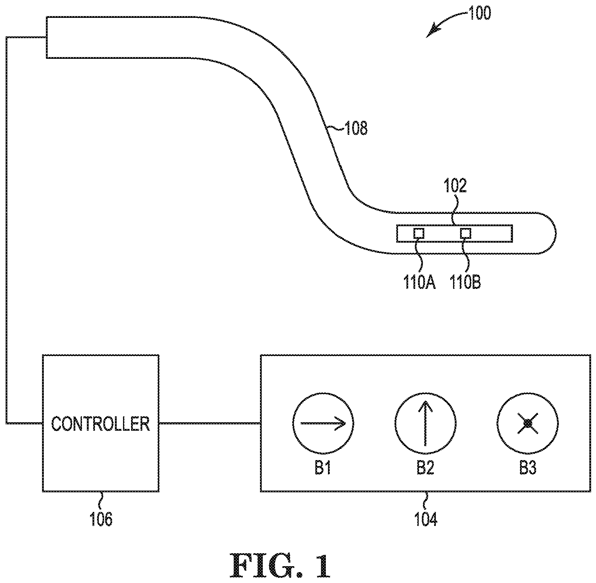

FIG. 1 is a diagram illustrating a tracking system 100 including a sensor assembly 102, magnetic field generator 104, a controller 106, and a probe 108 (e.g., catheter, imaging probe, diagnostic probe). The sensor assembly 102 can be positioned within the probe 108, for example, at a distal end of the probe 108. The tracking system 100 is configured to determine the location and orientation of the sensor assembly 102 and, therefore, the probe 108. Magnetic fields generated by the magnetic field generator 104 provide a frame of reference for the tracking system 100 such that the location and orientation of the sensor assembly 102 is determined relative to the generated magnetic fields. The tracking system 100 can be used in a medical procedure, where the probe 108 is inserted into a patient and the sensor assembly 102 is used to assist with tracking the location of the probe 108 in the patient.

The sensor assembly 102 is communicatively coupled to the controller 106 by a wired or wireless communications path such that the controller 106 sends and receives various signals to and from the sensor assembly 102. The magnetic field generator 104 is configured to generate one or more magnetic fields. For example, the magnetic field generator 104 is configured to generate at least three magnetic fields B1, B2, and B3. Each of the magnetic fields B1, B2, and B3 is directed in a different direction, as indicated by arrows in FIG. 1. Magnetic field B1 is a magnetic field in the horizontal direction, magnetic field B2 is a magnetic field in the vertical direction, and magnetic field B3 is a magnetic field into the page of FIG. 1. The controller 106 is configured to control the magnetic field generator 104 via a wired or wireless communications path to generate one or more of the magnetic fields B1, B2, and B3 to assist with tracking the sensor assembly 102 (and therefore probe 108).

The sensor assembly 102 is configured to sense the generated magnetic fields and provide tracking signals indicating the location and orientation of the sensor assembly 102 in up to six degrees of freedom (i.e., x, y, and z measurements, and pitch, yaw, and roll angles). Generally, the number of degrees of freedom that a tracking system is able to track depends on the number of magnetic field sensors and magnetic field generators. For example, a tracking system with a single magnetic field sensor may not be capable of tracking roll angles and thus are limited to tracking in only five degrees of freedom (i.e., x, y, and z coordinates, and pitch and yaw angles). This is because a magnetic field sensed by a single magnetic field sensor does not change as the single magnetic field sensor is "rolled." As such, the sensor assembly 102 includes at least two magnetic field sensors, 110A and 110B. The magnetic field sensors can include sensors such as inductive sensing coils and/or various sensing elements such as magneto-resistive (MR) sensing elements (e.g., anisotropic magneto-resistive (AMR) sensing elements, giant magneto-resistive (GMR) sensing elements, tunneling magneto-resistive (TMR) sensing elements, Hall effect sensing elements, colossal magneto-resistive (CMR) sensing elements, extraordinary magneto-resistive (EMR) sensing elements, spin Hall sensing elements, and the like), giant magneto-impedance (GMI) sensing elements, and/or flux-gate sensing elements. In addition, the sensor assembly 102 and/or the probe 108 can feature other types of sensors, such as temperature sensors, ultrasound sensors, etc.

The sensor assembly 102 is configured to sense each of the magnetic fields B1, B2, and B3 and provide signals to the controller 106 that correspond to each of the sensed magnetic fields B1, B2, and B3. The controller 106 receives the signals from the sensor assembly 102 via the communications path and determines the position and location of the sensor assembly 102 and probe 108 in relation to the generated magnetic fields B1, B2, and B3.

The magnetic field sensors can be powered by voltages or currents to drive or excite elements of the magnetic field sensors. The magnetic field sensor elements receive the voltage or current and, in response to one or more of the generated magnetic fields, the magnetic field sensor elements generate sensing signals, which are transmitted to the controller 106. The controller 106 is configured to control the amount of voltage or current to the magnetic field sensors and to control the magnetic field generators 104 to generate one or more of the magnetic fields B1, B2, and B3. The controller 106 is configured to receive the sensing signals from the magnetic field sensors and to determine the location and orientation of the sensor assembly 102 (and therefore probe 108) in relation to the magnetic fields B1, B2, and B3. The controller 106 can be implemented using firmware, integrated circuits, and/or software modules that interact with each other or are combined together. For example, the controller 106 may include computer-readable instructions/code for execution by a processor. Such instructions may be stored on a non-transitory computer-readable medium and transferred to the processor for execution. In some embodiments, the controller 106 can be implemented in one or more application-specific integrated circuits and/or other forms of circuitry suitable for controlling and processing magnetic tracking signals and information.

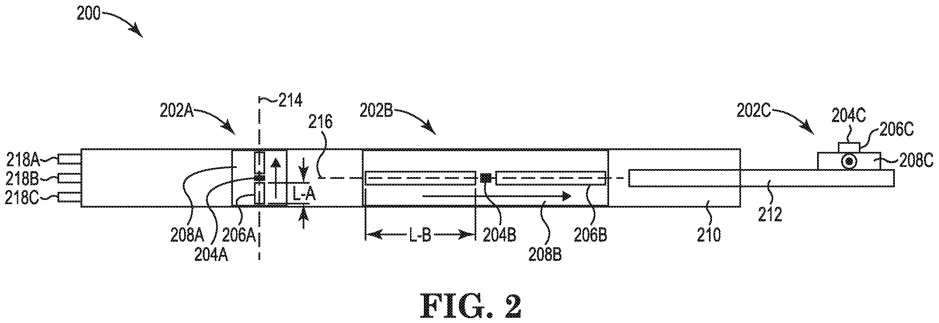

FIG. 2 shows a sensor assembly 200 that can be used in tracking systems such as the tracking system 100 of FIG. 1 and positioned in a probe like the probe 108 of FIG. 1. The sensor assembly 200 is shown as having three magnetic field sensors (i.e., a first magnetic field sensor 202A, a second magnetic field sensor 202B, and a third magnetic field sensor 202C), although is appreciated that the sensor assembly 200 could have only two magnetic field sensors or more than three magnetic field sensors. Moreover, the magnetic field sensors 202A-C can be ordered in different arrangement. In some embodiments, the sensor assembly 200 is part of a flex circuit assembly.

FIG. 2 shows each magnetic field sensor 202A-C as including an MR sensing element: a first MR sensing element 204A, a second MR sensing element 204B, and a third MR sensing element 204C. The MR sensing elements can be GMR sensing elements, TMR sensing elements, hall-effect sensing elements, CMR sensing elements, EMR sensing elements, spin-hall sensing elements, and the like. The MR sensing elements are configured to sense magnetic fields, like those generated by the magnetic field generator 104 of FIG. 1, and generate a responsive sensing signal.

Each magnetic field sensor 202A-C also includes at least one elongated magnetic field sensor component: 206A, 206B, and 206C. In some embodiments, the elongated magnetic field sensor components are flux guides. Flux guides can be configured to collect and direct magnetic flux to an MR sensing element, which senses generated magnetic fields and generates sensing signals indicative of the sensed generated magnetic fields.

The first magnetic field sensor 202A includes a first sensor substrate 208A, the second magnetic field sensor 202B includes a second sensor substrate 208B, and the third magnetic field sensor 202C includes a third sensor substrate 208C. In some embodiments, the MR sensing elements and elongated magnetic field sensor components are coupled to and/or positioned on the sensor substrates. The first magnetic field sensor 202A and the second magnetic field sensor 202B are shown being positioned on a common sensor assembly substrate 210, which can form part of a flex circuit. The third magnetic field sensor 202C is shown being positioned on another sensor assembly substrate 212, which is coupled to the common sensor assembly substrate 210, is oriented orthogonal to the common sensor assembly substrate 210, and can form part of a flex circuit.

As shown in the FIG. 2, the first magnetic field sensor 202A includes multiple flux guides 206A, which are positioned adjacent to the MR sensing element 204A. The flux guides 206A have a length, L-A, along an axis 214 that is perpendicular to a longitudinal axis 216 of the sensor assembly 200. When implemented in a probe, such as probe 108 of FIG. 1, the longitudinal axis 214 of the sensor assembly 200 can be parallel with a longitudinal axis of the probe. The flux guides 206B in the second magnetic field sensor 202B are positioned adjacent to the second MR sensing element 204B. The flux guides 206B have a length, L-B, in a direction along the longitudinal axis 214 of the sensor assembly 200. As shown in FIG 2, the length, L-B, of the flux guides 206B of the second magnetic field sensor 202B is longer than the length, L-A, of the first magnetic field sensor 202A. As a result, the second magnetic field sensor 202B has an overall length that is longer than an overall length of the first magnetic field sensor 202A. The longer flux guides 206B direct more magnetic flux to the second MR sensing element 204B compared to the amount of magnetic flux directed to the first MR sensing element 204A by the shorter flux guides 206A. As such, the second magnetic field sensor 202B can be characterized as having a greater sensitivity to magnetic fields than the first magnetic field sensor's sensitivity. In other words, for a given magnetic field amplitude, the second magnetic field sensor 202B generates a larger responsive sensing signal, which is used to determine location and orientation of the sensor assembly 200. This increased sensitivity is due to increased flux directed by the longer flux guides 206B to the second MR sensing element 204B.

The longer flux guides 206B of the second magnetic field sensor 202B take advantage of the geometry of a probe in that the flux guides 206B (and therefore second magnetic field sensor 202B) are designed to be longer in a longitudinal axis of a probe to provide increased sensitivity compared to the other magnetic field sensors. The increased sensitivity of the second magnetic field sensor 202B relative to the sensitivity of the other magnetic field sensors increases sensing performance of the overall sensor assembly 200. As mentioned above, a single sensor can be configured to sense up to five degrees of freedom (i.e., x, y, z, pitch, and yaw). Because the second magnetic field sensor 202B is more sensitive than the other magnetic field sensors of the sensor assembly 200, the sensed signals generated by the second magnetic field sensor 202B can dominate the sensed signals generated by the other magnetic field sensors. As such, the sensitivity of the first and third magnetic field sensors, 202A and 202C, need not be as sensitive as the second magnetic field sensor 202B and therefore can be made smaller, which can reduce the overall size of the sensor assembly 200. In some embodiments, the more sensitive magnetic field sensor can be two to ten times more sensitive than the other magnetic field sensors. In some embodiments, the more sensitive magnetic field sensor can be up to five times more sensitive than the other magnetic field sensors.

The sensing signals generated by the magnetic field sensors 202A-C can be transmitted from the sensing assembly 200 to a controller, such as the controller 106 of FIG. 1, wirelessly or via one or more conductors. FIG. 2 shows three conductors, 218A, 218B, and 218C, each corresponding to one of the magnetic field sensors, 202A, 202B, and 202C.

Like the sensor assembly 200 of FIG. 2, the sensor assembly embodiments described below utilize multiple magnetic field sensors with at least one of the sensors being more sensitive than the other magnetic field sensors. Such sensor assemblies take advantage of the geometry of probes such as catheters. Although the sensor assembly embodiments described below as being used with a probe, it is appreciated that the sensor assemblies can be utilized in other environments.

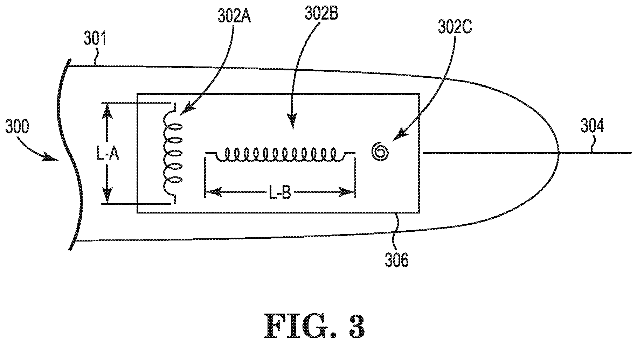

FIG. 3 shows a sensor assembly 300 that can be used in tracking systems such as the tracking system 100 of FIG. 1. The sensor assembly 300 is shown being positioned at a distal end of a probe 301, like the probe 108 of FIG. 1. The sensor assembly 300 is shown as having three magnetic field sensors (i.e., a first magnetic field sensor 302A, a second magnetic field sensor 302B, and a third magnetic field sensor 302C), although is appreciated that the sensor assembly 300 could have only two magnetic field sensors or more than three magnetic field sensors. Moreover, the magnetic field sensors 302A-C can be ordered in different arrangements. The magnetic field sensors in FIG. 3 are inductive sensing coils, which are arranged orthogonally to each other.

As shown in the FIG. 3, the first magnetic field sensor 302A extends a length, L-A, in a direction that is perpendicular to a longitudinal axis 304 of the probe 301. The second magnetic field sensor 302B extends a length, L-B, in a direction parallel to the longitudinal axis 304. The length, L-B, of the second magnetic field sensor 302B is longer than the length, L-A, of the first magnetic field sensor 302A. In some embodiments, the second magnetic field sensor 302B is at least 1.25 times longer than the first magnetic field sensor 302A. In some embodiments, the second magnetic field sensor 302B is at least twice as long as the first magnetic field sensor 302A. In some embodiments, the second magnetic field sensor 302B is at or between 1.25-2 times longer than the first magnetic field sensor 302A.

As a result of being longer, the second magnetic field sensor 302B can be characterized as having a greater sensitivity to magnetic fields than the first magnetic field sensor's sensitivity. In other words, for a given magnetic field amplitude, the second magnetic field sensor 302B generates a larger responsive sensing signal, which is used to determine location and orientation of the sensor assembly 300. The magnetic field sensors 302A-C can be coupled together and oriented relative to each other by various means 306. In some embodiments, the coupling and orientation means 306 includes suspending the magnetic field sensors 302A-C in an epoxy, potting, and the like. In some embodiments, the coupling and orientation means 306 includes using injection molding to couple the various sensors. The sensing signals generated by the magnetic field sensors 302A-C can be transmitted from the sensing assembly 300 to a controller, such as the controller 106 of FIG. 1, wirelessly or via one or more conductors.

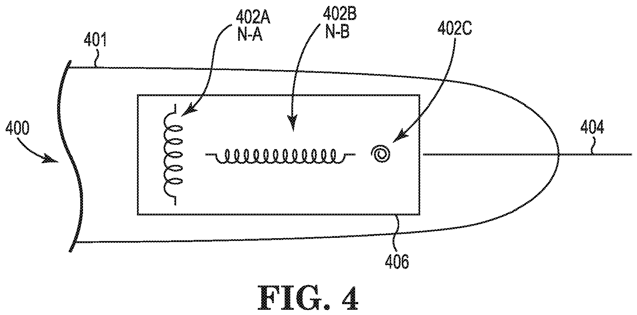

FIG. 4 shows a sensor assembly 400 that can be used in tracking systems such as the tracking system 100 of FIG. 1. The sensor assembly 400 is shown being positioned at a distal end of a probe 401, like the probe 108 of FIG. 1. The sensor assembly 400 is shown as having three magnetic field sensors (i.e., a first magnetic field sensor 402A, a second magnetic field sensor 402B, and a third magnetic field sensor 402C), although is appreciated that the sensor assembly 400 could have only two magnetic field sensors or more than three magnetic field sensors. Moreover, the magnetic field sensors 402A-C can be ordered in different arrangements. The magnetic field sensors in FIG. 4 are inductive sensing coils, which are arranged orthogonally to each other.

As shown in the FIG. 4, the first magnetic field sensor 402A extends in a direction that is perpendicular to a longitudinal axis 404 of the probe 401 and has a number of windings, N-A. The second magnetic field sensor 402B extends a length in a direction parallel to the longitudinal axis 404 and has a number of windings, N-B. The second magnetic field sensor 402B has a greater number of windings, N-B than the number of windings, N-A, of the first magnetic field sensor 402A. In some embodiments, the second magnetic field sensor 302B includes at least 1.25 times more windings than the first magnetic field sensor 302A. In some embodiments, the second magnetic field sensor 302B includes at least two times more windings than the first magnetic field sensor 302A. In some embodiments, the second magnetic field sensor 302B includes 1.25-2 times more windings than the first magnetic field sensor 302A.

As a result of having more windings, the second magnetic field sensor 402B can be characterized as having a greater sensitivity to magnetic fields than the first magnetic field sensor's sensitivity. In other words, for a given magnetic field amplitude, the second magnetic field sensor 402B generates a larger responsive sensing signal, which is used to determine location and orientation of the sensor assembly 400. The magnetic field sensors 402A-C can be coupled together and oriented relative to each other by various means 406. In some embodiments, the coupling and orientation means 406 includes suspending the magnetic field sensors 402A-C in an epoxy, potting, and the like. In some embodiments, the coupling and orientation means 306 includes using injection molding to couple the various sensors. The sensing signals generated by the magnetic field sensors 402A-C can be transmitted from the sensing assembly 400 to a controller, such as the controller 106 of FIG. 1, wirelessly or via one or more conductors.

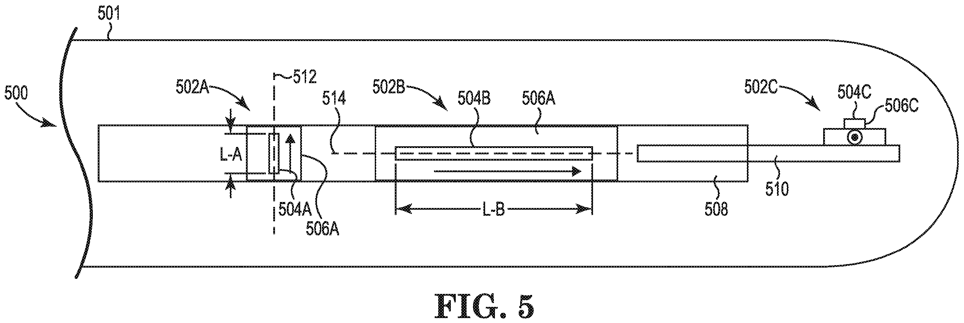

FIG. 5 shows a sensor assembly 500 that can be used in tracking systems such as the tracking system 100 of FIG. 1. The sensor assembly 500 is shown being positioned at a distal end of a probe 501, like the probe 108 of FIG. 1. The sensor assembly 500 is shown as having three magnetic field sensors (i.e., a first magnetic field sensor 502A, a second magnetic field sensor 502B, and a third magnetic field sensor 502C), although is appreciated that the sensor assembly 500 could have only two magnetic field sensors or more than three magnetic field sensors. Moreover, the magnetic field sensors 502A-C can be ordered in different arrangements. In some embodiments, the sensor assembly 500 is part of a flex circuit assembly.

Each magnetic field sensor in FIG. 5 includes at least one elongated magnetic field sensor component, 504A, 504B, and 504C, which can be AMR sensing elements. The first magnetic field sensor 502A includes a first sensor substrate 506A, the second magnetic field sensor 502B includes a second sensor substrate 506B, and the third magnetic field sensor 502C includes a third sensor substrate 506C. Each elongated magnetic field sensor component can be coupled to or positioned on its respective sensor substrate. The first magnetic field sensor 502A and the second magnetic field sensor 502B are shown being positioned on a common sensor assembly substrate 508, which can form part of a flex circuit. The third magnetic field sensor 502C is shown being positioned on another sensor assembly substrate 510, which is coupled to the common sensor assembly substrate 508, is oriented perpendicular to the common sensor assembly substrate 508, and can form part of a flex circuit.

As shown in the FIG. 5, the AMR sensing elements, 504A-C, each has an associated length: L-A, L-B, etc. The first AMR sensing element 504A has a length, L-A, along an axis 512 that is perpendicular to a longitudinal axis 514 of the probe 501. The second AMR sensing element 504B in the second magnetic field sensor 502B has a length, L-B, along the longitudinal axis 514 of the probe 501. The length, L-B, of the second AMR sensing element 504B is longer than the length, L-A, of the first AMR sensing element 504A. As a result, the second magnetic field sensor 502B has an overall length that is longer than an overall length of the first magnetic field sensor 502A. Because of the longer AMR sensing element 504B, the second magnetic field sensor 502B can be characterized as having a greater sensitivity to magnetic fields than the first magnetic field sensor's sensitivity. In other words, for a given magnetic field amplitude, the second magnetic field sensor 502B generates a larger responsive sensing signal, which is used to determine location and orientation of the sensor assembly 500.

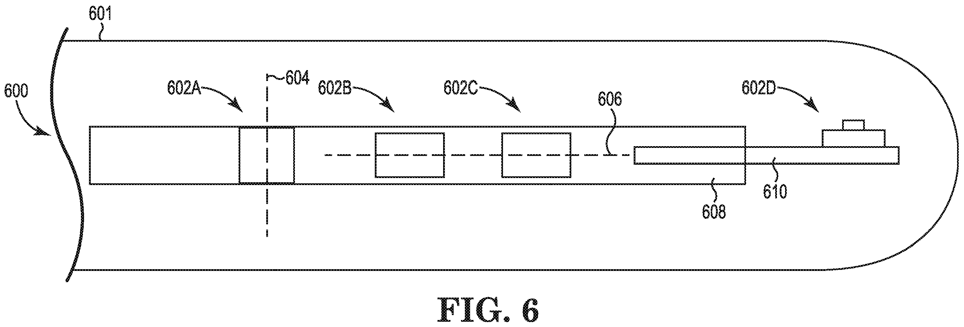

FIG. 6 shows a sensor assembly 600 that can be used in tracking systems such as the tracking system 100 of FIG. 1. The sensor assembly 600 is shown being positioned at a distal end of a probe 601, like the probe 108 of FIG. 1. The sensor assembly 600 is shown as having four magnetic field sensors (i.e., a first magnetic field sensor 602A, a second magnetic field sensor 602B, a third magnetic field sensor 602C, and a fourth magnetic field sensor 602D), although is appreciated that the sensor assembly 600 could have only two magnetic field sensors or more than three magnetic field sensors. Moreover, the magnetic field sensors 602A-C can be ordered in different arrangements. In some embodiments, the sensor assembly 600 is part of a flex circuit assembly.

The magnetic field sensors 602A-D can include sensors such as inductive sensing coils and/or various sensing elements such as MR sensing elements (e.g., AMR sensing elements, GMR sensing elements, TMR sensing elements, Hall effect sensing elements, CMR sensing elements, EMR sensing elements, spin Hall sensing elements, and the like), GMI sensing elements, and/or flux-gate sensing elements.

FIG. 6 shows the first magnetic field sensor 602A positioned and oriented such that its primary sensing direction is along an axis 604 that is perpendicular to a longitudinal axis 606 of the probe 601. The second magnetic field sensor 602B is positioned and oriented such that its primary sensing direction is parallel to the longitudinal axis 606 of the probe 601. Similarly, the third magnetic field sensor 602C is positioned and oriented such that its primary sensing direction is parallel to the longitudinal axis 606 of the probe 601. The fourth magnetic field sensor 602D is positioned and oriented along an axis that is orthogonal to the longitudinal axis 606 of the probe 601. Although the first, second, and third magnetic field sensors 602A-C are shown as being positioned on common substrate 608, it is appreciated that the magnetic field sensors can be positioned on individual substrates, common substrates, or suspended in epoxy, potting, etc., without a substrate, and/or a combination of the same. The fourth magnetic field sensor 602D is shown being positioned on another sensor assembly substrate 610, which is coupled to the common sensor assembly substrate 608, is oriented perpendicular to the common sensor assembly substrate 608, and can form part of a flex circuit.

Because the primary sensing direction of the second and third magnetic field sensors, 602B and 602C, are oriented along the same axis, the two magnetic field sensors can provide greater sensitivity compared to the sensitivity of the first and fourth magnetic field sensors, 602A and 602D. In other words, for a given magnetic field amplitude, the second magnetic field sensor 202B and the third magnetic field sensor 202C generate a combined larger responsive sensing signal compared to the other magnetic field sensor's responsive sensing signals.

It should be noted that, for simplicity and ease of understanding, the elements described above and shown in the figures are not drawn to scale and may omit certain features. As such, the drawings do not necessarily indicate the relative sizes of the elements or the non-existence of other features.

## Claims

1. A sensor assembly (200) comprising:
a first magnetic field sensor (202A) including a first substrate (208A) and a first elongated magnetic field sensor component (206A) extending a first length; and
a second magnetic field sensor (202B) including a second substrate (208B) and a second elongated magnetic field sensor component (206B) extending a second length that is shorter than the first length,
wherein the first magnetic field sensor (202A) has a greater sensitivity to a sensed magnetic field than the second magnetic field sensor (202B),
wherein the first elongated magnetic field sensor component (206A) is a first flux guide, and wherein the second elongated magnetic field sensor component (206B) is a second flux guide,
wherein the first magnetic field sensor (202A) includes a first magneto-resistive sensing element (204A) positioned adjacent the first flux guide, and wherein the second magnetic field sensor (202B) includes a second magneto-resistive sensing element (204B) positioned adjacent the second flux guide.

2. The sensor assembly (200) of claim 1, wherein the first and second magneto-resistive sensing elements (204A, 204B) are one of giant-magneto-resistive sensing elements, tunneling-magneto-resistive sensing elements, hall-effect sensing elements, colossal magneto-resistive sensing elements, extraordinary magneto-resistive sensing elements, and spin hall sensing elements.

3. The sensor assembly (200) of any of claims 1-2, wherein the first magnetic field sensor (202A) is configured to sense a first component of a magnetic field along a first axis, wherein the second magnetic field sensor (202B) is configured to sense a second component of the magnetic field along a second axis.

4. The sensor assembly (200) of any of claims 1-3, wherein the first magnetic field sensor (202A) and the second magnetic field sensor (202B) are coupled to a common substrate.

5. The sensor assembly (200) of any of claims 1-4, further comprising:
a third magnetic field sensor (202C) including a third substrate (208C) and a third elongated magnetic field sensor component (206C) extending a third length that is shorter than the first length.

6. The sensor assembly (200) of any of claims 1-5, wherein the first magnetic field sensor (202A) is at least twice as sensitive as the second magnetic field sensor (202B).

7. A system (100) comprising:
a magnetic field generator (104) configured to generate a magnetic field; and
a probe (108) having a longitudinal axis and a sensor assembly (102) according to claim 1 positioned at or near a distal end of the probe (108).

8. The system (100) of claim 7, wherein the first magnetic field sensor (202A) is configured to sense a first component of the magnetic field along the longitudinal axis (216), wherein the second magnetic field sensor (202B) is configured to sense a second component of the magnetic field along a second axis, and wherein the first magnetic sensor (202A) has a greater sensitivity to the first component of the magnetic field than a sensitivity of the second magnetic field sensor (202B) with respect to the second component of the magnetic field.

9. The system (100) of any of claims 7-8, wherein the first magnetic field sensor (202A) includes a first magneto-resistive sensing element, wherein the second magnetic field sensor (202B) includes a second magneto-resistive sensing element, wherein the first magneto-resistive sensing element is longer than the second magneto-resistive sensing element.

10. The system (100) of any of claims 7-9, wherein the first and second magnetic field sensors (202A, 202B) are one of giant-magneto-resistive sensing elements, tunneling-magneto-resistive sensing elements, hall-effect sensing elements, colossal magneto-resistive sensing elements, extraordinary magneto-resistive sensing elements, and spin hall sensing elements.

11. The system (100) of any of claims 7-10, further comprising:
a third magnetic field sensor (202C) configured to sense a third component of the magnetic field along a third axis that is different than the longitudinal axis (216) and the second axis.

## Patentansprüche

1. Sensoranordnung (200), umfassend:
einen ersten Magnetfeldsensor (202A), umfassend ein erstes Substrat (208A) und eine erste längliche Magnetfeldsensorkomponente (206A), die sich über eine erste Länge erstreckt; und
einen zweiten Magnetfeldsensor (202B), umfassend ein zweites Substrat (208B) und eine zweite längliche Magnetfeldsensorkomponente (206B), die sich über eine zweite Länge erstreckt, die kürzer als die erste Länge ist,
wobei der erste Magnetfeldsensor (202A) eine größere Empfindlichkeit gegenüber einem erfassten Magnetfeld aufweist als der zweite Magnetfeldsensor (202B),
wobei die erste längliche Magnetfeldsensorkomponente (206A) eine erste Flussführung ist, und wobei die zweite längliche Magnetfeldsensorkomponente (206B) eine zweite Flussführung ist,
wobei der erste Magnetfeldsensor (202A) ein erstes magnetoresistives Sensorelement (204A) umfasst, das benachbart zu der ersten Flussführung positioniert ist, und wobei der zweite Magnetfeldsensor (202B) ein zweites magnetoresistives Sensorelement (204B) umfasst, das benachbart zu der zweiten Flussführung positioniert ist.

2. Sensoranordnung (200) nach Anspruch 1, wobei das erste und das zweite magnetoresistive Sensorelement (204A, 204B) eines von riesenmagnetoresistiven Sensorelementen, tunnelmagnetoresistiven Sensorelementen, Hall-Effekt-Sensorelementen, kolossal magnetoresistiven Sensorelementen, außergewöhnlich magnetoresistiven Sensorelementen und Spin-Hall-Sensorelementen sind.

3. Sensoranordnung (200) nach einem der Ansprüche 1-2, wobei der erste Magnetfeldsensor (202A) dazu ausgelegt ist, eine erste Komponente eines Magnetfelds entlang einer ersten Achse zu erfassen, wobei der zweite Magnetfeldsensor (202B) dazu ausgelegt ist, eine zweite Komponente des Magnetfelds entlang einer zweiten Achse zu erfassen.

4. Sensoranordnung (200) nach einem der Ansprüche 1-3, wobei der erste Magnetfeldsensor (202A) und der zweite Magnetfeldsensor (202B) an ein gemeinsames Substrat gekoppelt sind.

5. Sensoranordnung (200) nach einem der Ansprüche 1-4, ferner umfassend:
einen dritten Magnetfeldsensor (202C), umfassend ein drittes Substrat (208C) und eine dritte längliche Magnetfeldsensorkomponente (206C), die sich über eine dritte Länge erstreckt, die kürzer als die erste Länge ist.

6. Sensoranordnung (200) nach einem der Ansprüche 1-5, wobei der erste Magnetfeldsensor (202A) mindestens doppelt so empfindlich ist wie der zweite Magnetfeldsensor (202B).

7. System (100), umfassend:
einen Magnetfeldgenerator (104), dazu ausgelegt, ein Magnetfeld zu erzeugen; und
eine Sonde (108) mit einer Längsachse und einer Sensoranordnung (102) nach Anspruch 1, positioniert an oder nahe einem distalen Ende der Sonde (108).

8. System (100) nach Anspruch 7, wobei der erste Magnetfeldsensor (202A) dazu ausgelegt ist, eine erste Komponente des Magnetfelds entlang der Längsachse (216) zu erfassen, wobei der zweite Magnetfeldsensor (202B) dazu ausgelegt ist, eine zweite Komponente des Magnetfelds entlang einer zweiten Achse zu erfassen, und wobei der erste Magnetsensor (202A) eine größere Empfindlichkeit gegenüber der ersten Komponente des Magnetfelds als eine Empfindlichkeit des zweiten Magnetfeldsensors (202B) gegenüber der zweiten Komponente des Magnetfelds aufweist.

9. System (100) nach einem der Ansprüche 7-8, wobei der erste Magnetfeldsensor (202A) ein erstes magnetoresistives Sensorelement umfasst, wobei der zweite Magnetfeldsensor (202B) ein zweites magnetoresistives Sensorelement umfasst, wobei das erste magnetoresistive Sensorelement länger als das zweite magnetoresistive Sensorelement ist.

10. System (100) nach einem der Ansprüche 7-9, wobei der erste und zweite Magnetfeldsensor (202A, 202B) einer von riesenmagnetoresistiven Sensorelementen, tunnelmagnetoresistiven Sensorelementen, Hall-Effekt-Sensorelementen, kolossal magnetoresistiven Sensorelementen, außergewöhnlich magnetoresistiven Sensorelementen und Spin-Hall-Sensorelementen sind.

11. System (100) nach einem der Ansprüche 7-10, ferner umfassend:
einen dritten Magnetfeldsensor (202C), dazu ausgelegt, eine dritte Komponente des Magnetfelds entlang einer dritten Achse zu erfassen, die sich von der Längsachse (216) und der zweiten Achse unterscheidet.

## Revendications

1. Assemblage de capteurs (200) comprenant :
un premier capteur de champ magnétique (202A) qui inclut un premier substrat (208A) et un premier composant de capteur de champ magnétique allongé (206A) qui est étendu sur une première longueur ; et
un deuxième capteur de champ magnétique (202B) qui inclut un deuxième substrat (208B) et un deuxième composant de capteur de champ magnétique allongé (206B) qui est étendu sur une deuxième longueur qui est plus courte que la première longueur ;
dans lequel le premier capteur de champ magnétique (202A) présente une sensibilité plus importante que le deuxième capteur de champ magnétique (202B) vis-à-vis d'un champ magnétique détecté ;
dans lequel le premier composant de capteur de champ magnétique allongé (206A) est un premier guide de flux, et dans lequel le deuxième composant de capteur de champ magnétique allongé (206B) est un second guide de flux ;
dans lequel le premier capteur de champ magnétique (202A) inclut un premier élément de détection magnéto-résistif (204A) qui est positionné de telle sorte qu'il soit adjacent au premier guide de flux ; et dans lequel le deuxième capteur de champ magnétique (202B) inclut un deuxième élément de détection magnéto-résistif (204B) qui est positionné de telle sorte qu'il soit adjacent au second guide de flux.

2. Assemblage de capteurs (200) selon la revendication 1, dans lequel les premier et deuxième éléments de détection magnéto-résistifs (204A ; 204B) sont des éléments de détection pris parmi les éléments de détection magnéto-résistifs géants, les éléments de détection magnéto-résistifs à effet tunnel, les éléments de détection à effet Hall, les éléments de détection magnéto-résistifs colossaux, les éléments de détection magnéto-résistifs extraordinaires et les éléments de détection à effet Hall et spin.

3. Assemblage de capteurs (200) selon l'une quelconque des revendications 1 et 2, dans lequel le premier capteur de champ magnétique (202A) est configuré pour détecter une première composante d'un champ magnétique suivant un premier axe, dans lequel le deuxième capteur de champ magnétique (202B) est configuré pour détecter une deuxième composante du champ magnétique suivant un deuxième axe.

4. Assemblage de capteurs (200) selon l'une quelconque des revendications 1 à 3, dans lequel le premier capteur de champ magnétique (202A) et le deuxième capteur de champ magnétique (202B) sont couplés à un substrat commun.

5. Assemblage de capteurs (200) selon l'une quelconque des revendications 1 à 4, comprenant en outre :
un troisième capteur de champ magnétique (202C) qui inclut un troisième substrat (208C) et un troisième composant de capteur de champ magnétique allongé (206C) qui est étendu sur une troisième longueur qui est plus courte que la première longueur.

6. Assemblage de capteurs (200) selon l'une quelconque des revendications 1 à 5, dans lequel le premier capteur de champ magnétique (202A) est au moins deux fois plus sensible que le deuxième capteur de champ magnétique (202B).

7. Système (100) comprenant :
un générateur de champ magnétique (104) qui est configuré pour générer un champ magnétique ; et
une sonde (108) qui comporte un axe longitudinal et un assemblage de capteurs (102) selon la revendication 1 qui est positionné au niveau ou à proximité d'une extrémité distale de la sonde (108).

8. Système (100) selon la revendication 7, dans lequel le premier capteur de champ magnétique (202A) est configuré pour détecter une première composante du champ magnétique suivant l'axe longitudinal (216), dans lequel le deuxième capteur de champ magnétique (202B) est configuré pour détecter une deuxième composante du champ magnétique suivant un deuxième axe, et dans lequel le premier capteur de champ magnétique (202A) présente une sensibilité plus importante vis-à-vis de la première composante du champ magnétique qu'une sensibilité du deuxième capteur de champ magnétique (202B) vis-à-vis de la deuxième composante du champ magnétique.

9. Système (100) selon l'une quelconque des revendications 7 et 8, dans lequel le premier capteur de champ magnétique (202A) inclut un premier élément de détection magnéto-résistif, dans lequel le deuxième capteur de champ magnétique (202B) inclut un deuxième élément de détection magnéto-résistif, dans lequel le premier élément de détection magnéto-résistif est plus long que le deuxième élément de détection magnéto-résistif.

10. Système (100) selon l'une quelconque des revendications 7 à 9, dans lequel les premier et deuxième capteurs de champ magnétique (202A, 202B) sont des éléments de détection pris parmi les éléments de détection magnéto-résistifs géants, les éléments de détection magnéto-résistifs à effet tunnel, les éléments de détection à effet Hall, les éléments de détection magnéto-résistifs colossaux, les éléments de détection magnéto-résistifs extraordinaires et les éléments de détection à effet Hall et spin.

11. Système (100) selon l'une quelconque des revendications 7 à 10, comprenant en outre :
un troisième capteur de champ magnétique (202C) qui est configuré pour détecter une troisième composante du champ magnétique suivant un troisième axe qui est différent de l'axe longitudinal (216) et du deuxième axe.
